# EUROPEAN PATENT SPECIFICATION

(11) **EP 3 842 092 B1**
(45) Date of publication and mention of the grant of the patent: **26.01.2022**
(21) Application number: 19219593.1
(22) Date of filing: 24.12.2019
(51) Int. Cl.: A61M 35/00, A61H 33/14, A61H 35/00

(54) **A DEVICE FOR TRANSCUTANEOUS APPLICATION OF CARBON DIOXIDE**
VORRICHTUNG ZUR TRANSKUTANEN ANWENDUNG VON KOHLENDIOXID
DISPOSITIF POUR APPLICATION TRANSCUTANÉE DE DIOXYDE DE CARBONE

(43) Date of publication of application: 30.06.2021
(73) Proprietor: Derma art d.o.o., 8250 Brezice (SI)
(72) Inventor: Truden, Maja, 4000 Kranj (SI); Vezoja, Daniel, 1230 Domzale (SI)
(74) Representative: Patentni Biro AF d.o.o.

(56) References cited:
- WO-A1-2018/142171
- DE-U1- 20 307 743
- US-A1- 2011 040 239
- US-A1- 2013 079 703
- US-B1- 9 480 830

## Description

### Field of the invention

The present invention belongs to the field of medical devices for treatment with carbon dioxide, more precisely to the field of devices for transcutaneous application of carbon dioxide. The invention also belongs to the field of piping, connections and valves for medical devices and medical use. The invention relates to a device for transcutaneous application of carbon dioxide for treatment of chronic wounds and/or neuropathy.

### Background of the invention and the technical problem

CO₂ can be applied to treat a variety of disorders, mostly treatment for peripheral vascular disorders (Dogliotti et al, 2011, Int Angiol. 30(1):12-7.). The benefits of bathing in CO₂-enriched water have been described (Hartmann et al, 1997, https://doi.org/10.1177/000331979704800406; Toriyama et al., 2002, Int Angiol. 21(4):367-73). It has been observed that CO₂ stimulates blood flow and microcirculation to increase partial O₂ pressure in local tissue, which is known as the Bohr effect (Irie et al, 2005, Circulation 111:1523-1529; Bohr et al., 1904, https://doi.org/10.1111/j.1748-1716.1904.tb01382.x). The Bohr effect indeed occurs in human body after transcutaneous administration of CO₂ as shown by Sakai et al. (2011, PloS One, 6, 9: e24137). More recently, CO₂ therapy has been found to induce mitochondrial apoptosis in human tumours, hence it has also been suggested for clinical testing for treatment of primary tumours (Oe et al, 2011, Biochem Biophys Res Commun 407: 148-152; Onishi et al, 2012, https://doi.org/10.1371/journal.pone.0049189; Takeda et al, 2014, PLoS One 9: e100530; Ueha et al, 2107, https://doi.org/10.3892/or.2017.5591).

Usually, CO₂ therapy is performed by bathing in CO₂ enriched water or by injection, wherein transcutaneous CO₂ application using 100% CO₂ gas is supported by applying CO₂ absorption-enhancing hydrogel (Onishi et al, 2012). Both known methods have disadvantages. CO₂ enriched water is prepared by supplying CO₂ in gaseous form from tanks, however the amount of CO₂ in water is small, due to potential danger of inhaling toxic amounts of CO₂. It has been estimated that the concentration of CO₂ in the enriched water is only 0.1 % and there is no evidence showing absorption in human body (Hashimoto et al, 2004, J Appl Physiol 96:226-232; Yamamoto et al, 2007, Int J Biometeorol 51:201-208). Further, such bathing is not suitable for hospital treatments and for patients with chronic or acute wounds.

A chronic wound is a wound that does not heal in a usual manner and it is widely accepted that wounds that do not heal within three months are considered chronic. Chronic wounds have different causes (ischemic, neuropathic, etc.) and may differ in the stage of healing in which they are detained. In some cases, such wounds may never heal or take years to do so. Delayed wound healing has also been linked to peripheral neuropathy, which is a condition where peripheral nerves are damaged and cause various unpleasant sensations, including pain. Peripheral neuropathy can be a result of several different causes - traumatic injuries, infections, metabolic problems, inherited causes and exposure to toxins, however one of the most common causes is diabetes. In patients with this metabolic condition, nerve damage tends to lead to a loss of sensation in limbs, usually feet. Wounds on the lower extremities are often overlooked, resulting in delayed wound care and untreated infection that, if it turns gangrenous, may need to be amputated to stop the spread.

Amputations could be decreased, limited or even prevented by improving blood flow and circulation in affected areas. As carbon dioxide has been known to exert these desired effects, there is a need for a device for transcutaneous application of carbon dioxide, which will allow safe treatment of neuropathy or chronic wounds without the need for bathing, injection or any supplemental hydrogel or water-based CO₂ carriers, which could deteriorate the state of wounds in diabetic patients. Thus, the technical problem solved by the present invention is construction of a device and all its parts, which will enable safe delivery of adequate amounts of CO₂ through the patient's skin for treatment.

### State of the art

Several different devices for trans- or subcutaneous delivery of CO₂ into human body are already known, but they use different approaches than the present invention. Mostly, the known solutions are for injection-based therapy (subcutaneous delivery). One such solution is disclosed at web page http://www.mbemedicale.it/en/prodotto/venusian-co2-therapy. This device is primarily intended for use in gynaecology for ultrasound scans where CO₂ is supplied into the abdominal cavity in order to separate the space between individual organs. Webpage available at the address https://www.alibaba.com/showroom/co2-carboxy-therapy-machine.html discloses a pen for cosmetic purposes that comprise CO₂ ampules. Such solutions do not deliver the needed amounts of CO₂ for successful treatments and the above described Bohr effect. Although subcutaneous CO₂ injections can deliver 100% CO₂, they are invasive, involve risks for infection and are only local, meaning that coverage of larger parts of human body is challenging.

On the other hand, Sakai et al (2011, PloS One, 6, 9: e24137) used a device for transcutaneous CO₂ delivery comprising a wrap for covering a part of the body and allowing supply of 100% CO₂ together with a hydrogel, so that the supplied CO₂ remained in the wrap. Similar approach was used by Ueha et al (2017; https://doi.org/10.3892/or.2017.5591), where transcutaneous administration of CO₂ to the area of skin around the tumour was achieved with a CO₂ hydrogel. The area was then sealed with a polyethylene bag, and 100% CO₂ gas was delivered into the bag.

The solution shown on the web page https://www.airjectorvet.com/ comprises a bag placed around the part of an animal that has to be treated with CO₂. The latter is introduced into the sealed bag with an especially adapted gun. After the treatment the CO₂ is released into the environment, which must be an open space to prevent intoxications with the gas. This solution differs from the present invention in many aspects, most importantly the gun does not allow introduction of concentrated CO₂ into the bag. Further, the present invention has a controlled release of CO₂ so that users of the device and the medical staff are safe.

Patent application WO2018142171 discloses an apparatus adapted for transcutaneous treatment by gas. The apparatus comprises a rigid-wall cabinet and having an upper opening and at least one openable panel, wherein a seat is arranged inside the cabinet, and the cabinet comprises at least one gas concentration measurement unit, and the cabinet further comprises a gas inlet for feeding the treatment gas and a gas outlet for discharging the treatment gas; a gas supply unit coupled to the gas inlet of the cabinet via a pipe and through an interposed controllable valve; and a gas tank connected to the gas supply unit. The apparatus further comprises a ventilation unit connected to the gas outlet of the cabinet through an interposed controllable valve, said ventilation unit being coupled via a further pipe to a space hermetically separated from the room containing the cabinet, and wherein said ventilation unit is designed so that it can discharge the entire volume of the treatment gas from the inner space of the cabinet within at most 5 seconds. The apparatus further comprises a control unit for receiving the signals of the gas concentration measurement unit and for controlling operation of the valves and said ventilation unit based on at least the signals of the gas concentration measurement unit.

Patent application US2013079703 describes a method of carbon dioxide gas treatment for improvement or promotion of circulation of blood in an ischemic region. The following steps (a) to (d) are continued at least once per day for four weeks, that is, a step (a) of pulverizing and dissolving carbon dioxide gas into a liquid, and producing a carbon dioxide gas mist by forming the same into a mist; a step (b) of spraying the carbon dioxide gas mist into a carbon dioxide gas mist-enclosing means for enclosing the living organism in an air tight state, a step (c) of expelling gas existing in the carbon dioxide gas mist-enclosing means into the outside, if necessary in parallel with the step (b), in order to maintain the pressure of gas within the carbon dioxide gas mist-enclosing means at or above a prescribed value being higher than the atmospheric pressure, and a step (d) of continuing such a step of supplying, for at least 20 minutes, the carbon dioxide mist into the carbon dioxide gas mist-enclosing means. These steps differ significantly form the present invention. Furthermore, the CO₂ is present in a relatively low amount and not even in gaseous state, thus sufficient sealing is not required. Suitable sealing is also not described.

Utility model DE20307743 discloses a carbon dioxide skin absorption treatment unit that a sack like element with a gas tight closure fixed to a folding box with regulator inlet from the CO₂ tank and gas warming and humidifying unit and inflatable pillow and bed. This solution is not for medical use as the device does not allow leak-free emptying of the unit, which may lead to significant amounts of CO₂ into air inhaled by the user.

The present invention solves the above-mentioned disadvantages of the known solutions.

### Description of the solution of the technical problem

The invention is intended for performing transcutaneous CO₂ application treatments of all kinds of chronic or acute wounds and conditions where the basic problem is insufficient blood supply and is defined in the appended claims. Transcutaneous CO₂ administration is enabled by law of diffusion from the part with high CO₂ concentration (chamber of the device) to the part with a lower CO₂ concentration (the patient's body or body part). By introducing significant amounts of CO₂ onto the patient's body or body part blood circulation is improved, as well as nutritive perfusion of the treated area, which is crucial for faster healing of chronic wounds and neuropathy.

The essence of the device for transcutaneous application of carbon dioxide for treatment of chronic wounds or neuropathy is in that the device comprises at least:
- a therapeutic chamber comprising at least a portion to receive a part of a patient's body to which the carbon dioxide is to be applied,
- an inlet/outlet pipe connecting the chamber with a CO₂ distribution system, wherein the inlet/outlet pipe is connected to the chamber with a suitable element or a valve;
- the CO₂ distribution system comprising a housing where at least the following components are installed:
   ∘ a first pipe with a first valve for suction of air out of the chamber,
   ∘ a second pipe with a second valve for supplying CO₂ from a tank/reservoir; wherein the first and second pipe are combined into the inlet/outlet pipe upstream of the valves;
   ∘ at least one device for ensuring air flow through the said valves and inlet/outlet pipe, preferably a ventilator or a pump;
   ∘ preferably at least one airflow measuring device for measuring the air flow through the valve on the inlet/outlet pipe or through any valve;
   ∘ preferably a reservoir for CO₂, where it is stored at the pressure of 1 to 5 bar, connected to the second pipe;
   ∘ an outlet pipe for leading the used air from the chamber through the wall to the external environment of the building where the device is installed; and
- at least one tank for storing CO₂ suitably connected to the CO₂ distribution system or preferably to the reservoir.

The outlet pipe is connected to the inlet/outlet pipe through the first pipe of the CO₂ distribution system.

The device can be further provided with a filter in the CO₂ distribution system to filter out any impurities and/or a silencer for decreasing the sound resulting from the flow of gas under pressure. Said filter is preferably installed in the pipe of the CO₂ distribution system between the ventilator and the inlet/outlet pipe. It is attached in any suitable way, usually with clamps. The silencer is preferably installed between the second valve and the ventilator. The CO₂ distributing system may have more pipes and valves, preferably one additional pipe with a valve is provided for delivering air to the chamber before it is filled with CO₂ in order to achieve CO₂ concentrations below 100%.

The device may be further equipped with suitable electronics for easier controlling and managing of the device, wherein the controller has suitable buttons connected with a control device that opens and closes the valves used in the CO₂ distribution system, so that any particular CO₂ concentration in the chamber may be achieved.

The chamber comprising at least a portion to receive a part of a patient's body to which the carbon dioxide is to be applied can be a flexible (soft) chamber such as a wrap or it can be designed as a chamber with supporting elements enabling a certain geometry of the chamber when it is filled with air and/or carbon dioxide. The chamber may have different sizes, wherein it can be appropriately small to receive only a foot, a part of a leg, a whole leg, an arm or a part of an arm or it can be bigger to accommodate the whole body of a patient with the exception of patient's head. Possible materials for the chamber are all biocompatible materials, which are not permeable to CO₂, wherein the preferred choice is polyethylene, especially low-density polyethylene. The chamber is preferably for single use for hygienic reasons to prevent possible transfer of infections.

The inlet/outlet pipe to the chamber is only one, thereby rendering the construction of the device simpler. Further, its maintenance is easier. The inlet/outlet pipe is coupled to the chamber with a suitable passage valve (the gate valve), preferably the valve is comprising a rotating part and a static part, the latter being adapted for introduction into the inlet/outlet pipe. The rotating part has two removable parts, which are screwed into place from the interior of the chamber. Thereby the passage valve allows safe supply and suction of air to and from the chamber. When the chamber is flexible (soft) as a wrap, the gate valve is necessary.

The device can be preferably equipped with the reservoir for storing CO₂ at pressure from 1 to 5 bar. The reservoir is connected to the gas tank, where the gas is stored at pressures around 50 bars. Presence of the reservoir allows faster filling of the chamber, as the gas is already in gaseous state, at a suitable temperature and at suitable pressure. Namely, a part of the gas is led from the gas tank to the reservoir, where it due to a larger space expands, thereby also warming without any heaters. Airtightness of the CO₂ distribution system inside the housing is ensured by using suitable seals and/or welding all metal components to each other, meaning that the valves are welded to the pipes, while the pipes are welded to the housing of the ventilator. The inlet/outlet pipe is connected to the system with a clamp and all attachments are provided with suitable seals, so that the CO₂ does not leak into the room where the device is used. The ventilator is preferably housed in a two-part housing welded together, wherein the housing is provided with a suitable number of holes for attachment of the required number of pipes. The valves may be any suitable, including electromagnetic or mechanic, controlled in any suitable way. The pipes are preferably metal, but can also be made of plastic materials, wherein welding is not possible but can be replaced with suitable seals such as rubber, silicon, Teflon and similar seals known in the art.

The outlet pipe for leading the used air from the chamber through the wall to the external environment of the building where the device is installed is preferably equipped with a telescopic transition for easy adjustment to a wide variety of walls, which often have different thicknesses. The telescopic transition may be extended or retracted depending on the wall, wherein its construction enables that it discharges the whole air from the chamber to the environment. The telescopic transition also seals the passage through the wall and prevents escape or return of the gas back into the room with the device according to the invention. The inner part of the telescopic transition may be provided with a seal, preferably installed on the flange of the inner part.

The examples and functioning method are left for illustrative purposes but do not form part of the present invention as they do not contain all the features of the claims.

A first preferred example of the device for transcutaneous application of carbon dioxide for treatment of chronic wounds or neuropathy is in that the device comprises:
- a therapeutic chamber comprising at least a portion to receive a part of a patient's body to which the carbon dioxide is to be applied,
- an inlet/outlet pipe connecting the chamber with a CO₂ distribution system;
- the CO₂ distribution system comprising a housing where at least the following components are installed:
   ∘ a first pipe with a first valve for suction of air out of the chamber, and a first airflow measuring device for measuring the air flow through the first valve,
   ∘ a second pipe with a second valve for supplying air to the chamber and a second air flow measuring device for measuring the air flow through the second valve,
   ∘ a third pipe with a third valve for supplying CO₂ from a tank or a reservoir and a third air flow measuring device for measuring the air flow through the third valve;
      wherein the first, second and third pipe are combined into the inlet/outlet pipe upstream of the valves;
   ∘ one ventilator, preferably two, for ensuring air flow through the said valves and inlet/outlet pipe,
   ∘ preferably a reservoir for CO₂, where the gas is stored at the pressure of 1 to 5 bar, connected to the third pipe;
   ∘ an outlet pipe for leading the used air from the chamber through the wall to the external environment of the building where the device is installed; and
- at least one tank for storing CO₂ suitably connected to CO₂ distribution system or preferably to the reservoir.

Said airflow measuring devices on individual pipes and valves may be replaced with one airflow measuring device installed on the inlet/outlet pipe and preferably also measuring the airflow through the gate valve. Every embodiment may be further equipped with a silencer and/or a filter and/or electronics for control. In case two ventilators are used, they are rotating in different directions with regards to the airflow (into the chamber or out of the chamber).

The second preferred example of the device for transcutaneous application of carbon dioxide for treatment of chronic wounds or neuropathy is in that the device comprises:
- a therapeutic chamber comprising at least a portion to receive a part of a patient's body to which the carbon dioxide is to be applied,
- an inlet/outlet pipe connecting the chamber with a CO₂ distribution system,
- the CO₂ distribution system comprising a housing where at least the following components are installed:
   ∘ a first pipe with a first valve for suction of air out of the chamber,
   ∘ a second pipe with a second valve for supplying CO₂ from a tank or reservoir;
      wherein the first and second pipe are combined into the inlet/outlet pipe upstream of the valves;
   ∘ a ventilator in a sealed housing for ensuring air flow through the said valves and inlet/outlet pipe,
   ∘ preferably a reservoir for CO₂, where the gas is stored at the pressure of 1 to 5 bar, connected to the second pipe;
   ∘ preferably a silencer for decreasing the sound resulting from the flow of gas under pressure;
   ∘ preferably a removable filter for filtering any impurities so that they do not reach the chamber;
   ∘ an outlet pipe for leading the used air from the chamber through the wall to the external environment of the building where the device is installed; and
- at least one tank for storing CO₂ suitably connected to the CO₂ distribution system or preferably to the reservoir.

Presence of at least two valves and suitable piping ensures that the carbon dioxide concentration is adapted to the needs of therapy.

The three valves (first, second, third) and at least one air flow measuring device in the first preferred embodiment enable controlling air composition inside the chamber, so that different CO₂ concentrations can be achieved. The preferred range of CO₂ concentration inside the chamber is 10 to 100 %, wherein most preferred range is between 30 and 90 %. For example, if a 30 % (V/V) concentration is needed inside the chamber and the total volume of the chamber is 100 L, 70 L of air will be supplied through the second valve and 30 L of CO₂ will be supplied through the third valve. A 90 % (V/V) concentration may be achieved by supplying 10 L of air through the second valve and 90 L of CO₂ through the third valve.

Preferably, the valves are controlled with a controller or a suitable computer/computer program in order to ensure correct CO₂ concentrations inside the chamber. The controller preferably has a button for emptying the chamber, which can turn on ventilator and open the first valve for air suction. All other valves are closed. When the chamber is completely empty, the ventilator is turned off and the first valve is closed. Then the actual volume of the chamber is determined by filling it with air, wherein information is obtained by measuring air flow. Based on the actual volume of the chamber the amount of air has to be pumped out and which amount of CO₂ has to be led into the chamber. When the chamber is full the operator stops filling by pressing a suitable button. After the therapy is done, a discharge button provided on the controller is pushed and this leads to the first valve being open and the second valve is closed, so that all air/CO₂ from the chamber is led to the exterior of the building.

The two valves and the airflow measuring device in the second preferred embodiment enable controlling of CO₂ concentrations inside the chamber so that the air is sucked from the chamber through the first pipe with the first valve and CO₂ is supplied from the tank or the reservoir to the chamber through the second pipe with the second valve. Thereby a CO₂ concentration near 100% is achieved in the chamber. If a lower concentration of CO₂ is required, the chamber is initially not emptied, but a certain amount of air may be left inside it. This embodiment has simpler operation and requires no electronics for control, although they are preferred.

The functioning method of the said device comprises the following steps:
- in case of flexible chamber, first attaching the chamber to the inlet-outlet pipe via the valve or the gate valve;
- placing at least a part of patient's body into the chamber and sealing the chamber;
- sucking out all air from the chamber with the first valve with a ventilator and leading all sucked air to the exterior of the building with the room where the device is used;
- leading CO₂ from the tank to the CO₂ distribution system or preferably the reservoir for allowing the CO₂ to expand in the reservoir, which results in decrease of its pressure in an increase of its temperature;
- closing the first valve and opening the second valve to supply a desired amount of air into the chamber and/or opening the third valve to supply a desired amount of 100% CO₂ into the chamber.

When the CO₂ administration is finished, the air with the CO₂ is sucked from the chamber through the outlet pipe and led outside of the building where the device is installed. When all air has been emptied from the chamber, it can be unsealed and removed, so that the patient may leave. Usually the therapy lasts for 10 minutes to up to 2 hours, wherein the length of each therapy can be adjusted based on the patient's state.

Use of the device is suitable for all patients with impaired microcirculation; especially but not limited to patients affected with: chronic and acute wounds, neuropathy, muscle tears, etc.

The invention has been tested on 47 patients (38 males and 9 females aged 65.4±12.0 years) with the total number of 61 diabetic wounds. Control group consisted of 26 patients (21 males and 5 females aged 66.5±10.7 years) with the total number of 31 diabetic wounds (median volume: 528 mm³, median area: 253 mm²), who underwent placebo treatment with transcutaneous application of air. Experimental group consisted of 21 patients (21 males and 5 females aged 66.5±10.7) with the total number of 30 diabetic wounds (median volume: 351 mm³, median area: 241 mm²), who underwent treatment with transcutaneous application of CO₂ using the invention. Testing lasted for 4 weeks and laser Doppler blood perfusion in foot skin microcirculation, heart rate and blood pressure measurements were carried out. Further, each subject underwent monofilament and vibration sensation tests. After the treatments following main findings were observed:
1) 67% (20 out of 30) of all the wounds from the experimental groups were successfully healed, whereas the volume and area of the unhealed wounds on average decreased by 96% and 89%, respectively. None of the wounds from the control group was healed.
2) The results of the monofilament and vibration tests showed statistically significant improvement in terms of the sites with perceived monofilament/vibration stimulus for the experimental group.
3) Results of laser Doppler blood perfusion indicate that the function of the endothelial and neurogenic vascular tone regulating mechanisms of microcirculation improved significantly for the experimental group.
4) Absolute values of heart rate and arterial blood pressure before and after CO₂ therapies indicate that no systemic effects were caused during the treatment.

The device for transcutaneous application of carbon dioxide for treatment of chronic wounds or neuropathy, will be described in further detail based on possible embodiments and figures, which show:
- Figure 1: An embodiment of the device according to the invention
- Figure 2: Detailed view of the device shown in figure 1
- Figure 3: Valves and piping inside the CO₂ distribution system connected to the chamber with the inlet/outlet pipe with two pipes (a) and three pipes (b)
- Figure 4: The gate valve for supplying gas into the chamber from the inlet/outlet pipe
- Figure 5: Installation of the gate valve shown in figure 4 into the chamber
- Figure 6: Outlet pipe with the telescopic intra-wall part
- Figure 7: installation of telescopic intra-wall part in the wall of a building

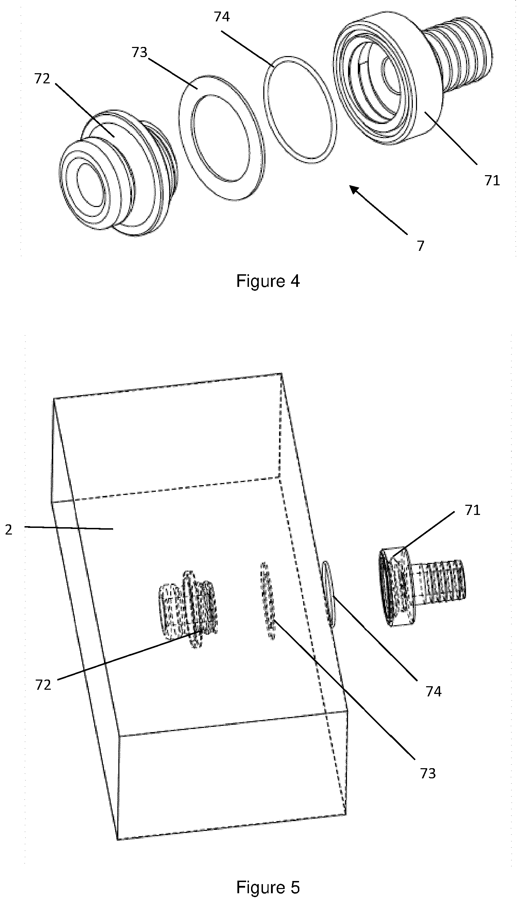

The Figures form part of the invention when the device comprises a gate valve as in the embodiment of Fig.4. Embodiments not comprising a gate valve as claimed in claim 1 do not form part of the invention.

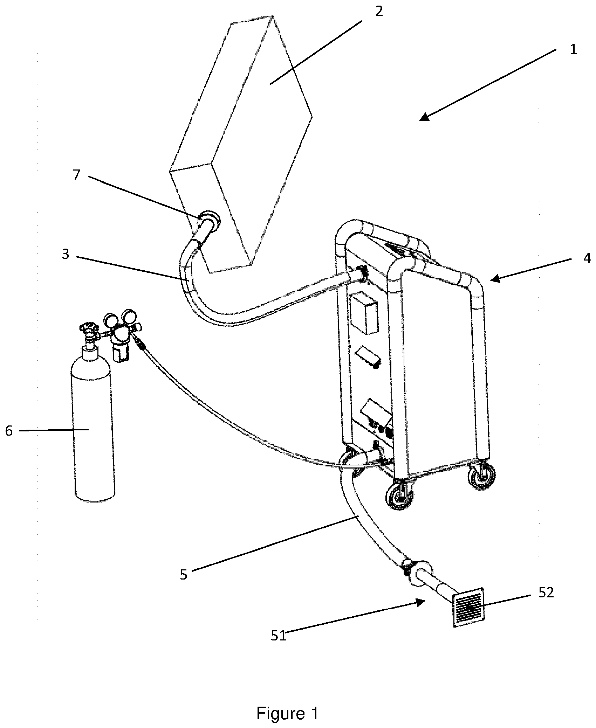

Figure 1 shows an embodiment of the device 1 according to the invention, the device 1 comprising the following:
- a therapeutic chamber 2 comprising at least a portion to receive a part of a patient's body to which the carbon dioxide is to be applied,
- one inlet-outlet pipe 3 connecting the chamber 2 with a CO₂ distribution system 4, wherein a gate valve 7 is preferably used, especially if the chamber 2 is flexible;
- the CO₂ distribution system 4;
- an outlet pipe 5 for leading the used air from the chamber 2 through a telescopic part 51 and an outlet 52 in a wall to the external environment of the building where the device is installed; and
- at least one tank 6 for storing CO₂ suitably connected to the CO₂ distribution system.

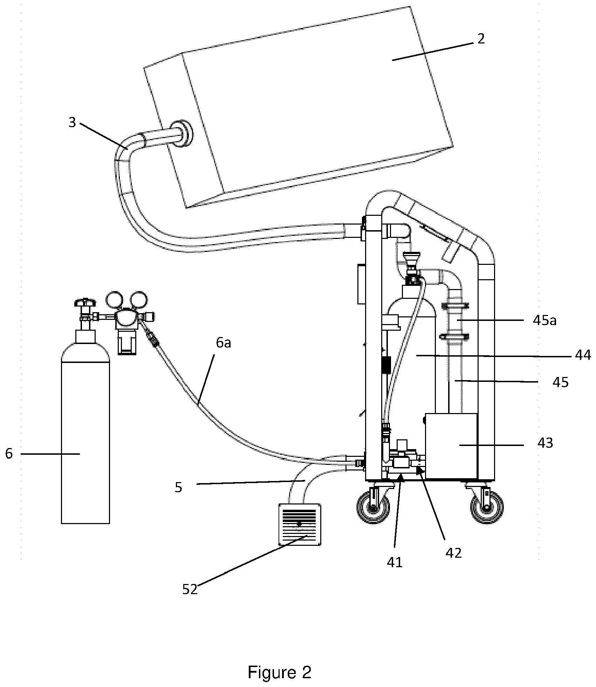

Figure 2 shows a detailed view of the preferred embodiment of the device, wherein CO₂ is provided from the tank into a reservoir 44 forming a part of the CO₂ distribution system. The CO₂ expands in the reservoir and can be then supplied to the chamber via the piping, valves and ventilator as described above. As said above, this enables that the CO₂ is at pressure 1 to 5 bar, which speeds up filling of the chamber. The CO₂ distribution system 4 has at least a first pipe 41 and a second pipe 42, connected to a ventilator 43, which is connected to the inlet/outlet pipe 3 with a pipe 45, which is preferably equipped with a filter 45a. When the CO₂ administration is finished, the CO₂ is led out through the first pipe 41 to the outlet pipe 5 into the exterior of the building.

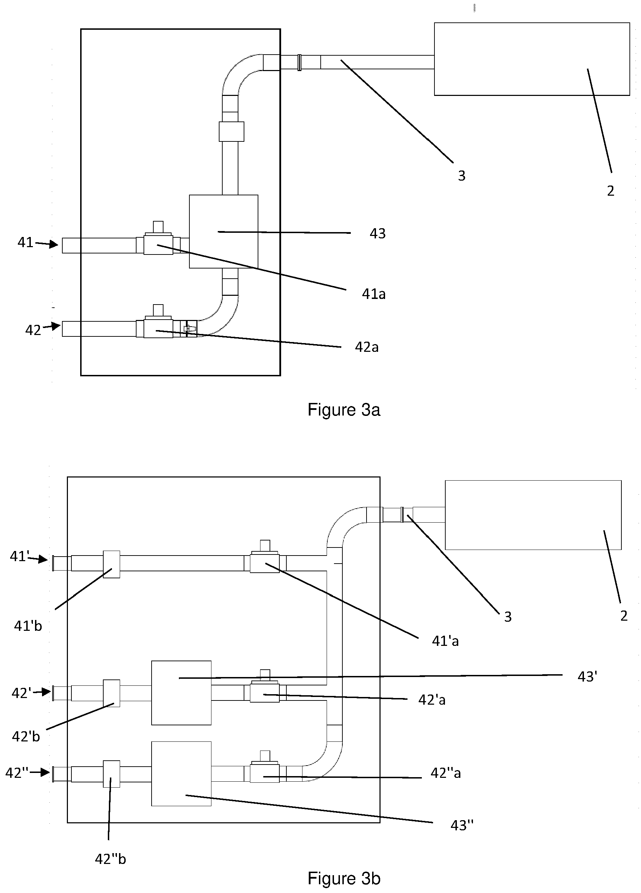

Figure 3a shows a possible embodiment of the CO₂ distribution system comprising a housing where two pipes 41,42 are installed, each pipe having its own valve 41a, 42a, and both pipes connected to the ventilator 43, which is then connected to the inlet/outlet pipe 3 and consequently the chamber 2.

Figure 3b shows the preferred embodiment of the CO₂ distribution system comprising a housing where the following components are installed:
- a first pipe 41' with a first valve 41'a for supplying CO₂ from the reservoir or the tank;
- a second pipe 42' with a second valve 42'a for supplying air from the environment;
- a third pipe 42" with a third valve 42"a for suction of air out of the chamber;
   wherein the said pipes are combined into the inlet/outlet pipe 3 upstream of the valves;
- two ventilators 43' and 43" for ensuring air flow through the said valves and inlet/outlet pipe,
- at least one airflow measuring device 41'b, 42'b, 42"b for measuring the air flow through the gate valve on the inlet/outlet pipe or through any valve;
- the reservoir for CO₂, where it is stored at the pressure of 1 to 5 bar, connected to the third pipe;

Pipes, valves, ventilator and the reservoir are connected airtightly with suitable welding and sealing. The valves may be any suitable valves such as electromagnetic or mechanical valves, wherein the gate valve has to be present in case the chamber is designed as a flexible wrap.

The gate valve 7 for connecting the inlet/outlet pipe 3 to the chamber 2 is shown in figure 4 and it comprises:
- a rotating part 72;
- preferably a washer 73;
- a seal 74; and
- a static part 71, the latter being adapted for introduction into the inlet/outlet pipe; wherein the rotating part has two removable parts, which are screwed into place from the interior of the chamber. The seal 74 is installed in the static part, while the washer prevents rotation of the chamber, when the rotating part is screwed into place with the static part 71. The washer is especially useful for flexible chambers, while it is not needed in hard chambers. Preferably the gate valve is made of biocompatible medical grade plastics. Figure 5 shows installation of the gate valve 7, wherein the rotating part 72 and the washer 73 are installed from the interior of the chamber, while the seal and the static part are installed from the direction of the inlet/outlet pipe 3. The rotating part 72 and the static part 71 have corresponding treads, which can interlock so that air leakage is prevented. This is further ensured with the seal 74.

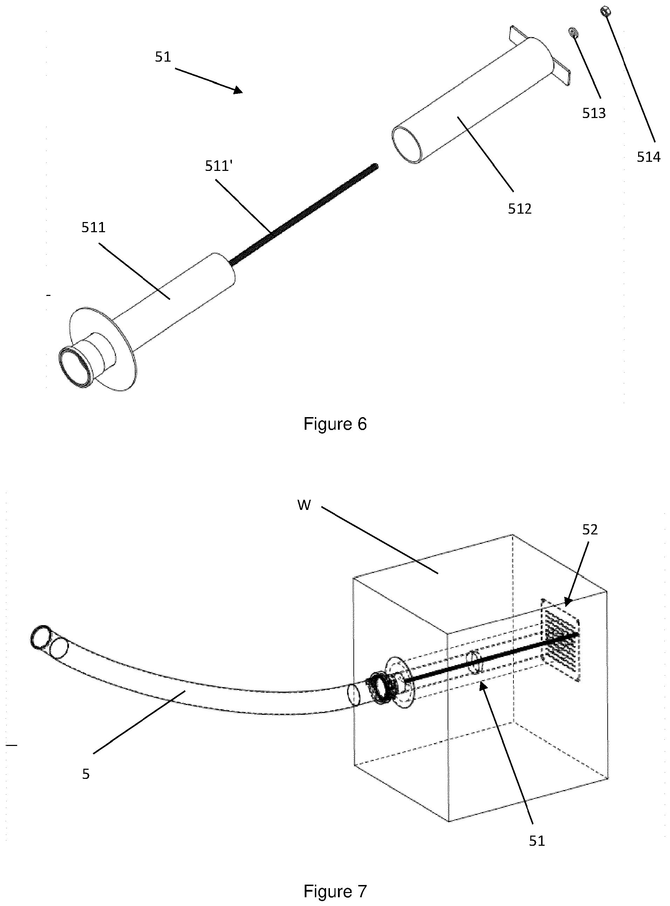

Figure 6 and 7 show the telescopic intra-wall part 51 and its installation into the wall W. The telescopic part 51 comprises:
- an inner part 511; the inner part's flange optionally provided with a seal for improved sealing
- an outer part 512;
- a washer 513; and
- a nut 514.

The wall has to be equipped with a pre-prepared hole having a diameter between 10 mm to 100m, into which the telescopic part 51 is installed. The smaller inner part 511 can move along the outer part 512, wherein a threaded pole 511' runs through the entire length of the telescopic part 51. A second nut is provided for tightening. The telescopic part can be adjusted so that it corresponds to the thickness of the wall W into which it is to be installed. The outlet 52 can be performed in any suitable way. Air can travel through the telescopic part into the interior of the CO₂ distribution system, more precisely towards the first pipe 41, or can travel to the exterior of the building when the therapy is over.

Within the scope of the invention as described herein and defined in the claims, other embodiments of the device for transcutaneous application of carbon dioxide for treatment of chronic wounds or neuropathy that are clear to person skilled in the art may be possible, which does not limit the essence of the invention as described herein and defined in the claims.

## Claims

1. A device (1) for transcutaneous application of carbon dioxide for treatment of chronic wounds or neuropathy comprising at least:
- a therapeutic chamber (2) comprising at least a portion to receive a part of a patient's body to which the carbon dioxide is to be applied,
- a CO₂ distribution system (4), comprising
o a first pipe (41) with a first valve (41a) for supplying CO₂ from a tank (6) or a reservoir,
o a second pipe (42) with a second valve (42a) for suction of air out of the chamber (2);
- at least one tank (6) for storing CO₂ suitably connected to the CO₂ distribution system (4) or preferably to a reservoir (44),
**characterized in that** the device (1) further comprises:
- an inlet/outlet pipe (3) connecting the chamber (2) with a CO₂ distribution system, wherein the inlet/outlet pipe is connected to the chamber (2) with a suitable element or a valve (7);
- and **in that** the CO₂ distribution system (4) comprises a housing :
wherein the first and second pipe are combined into the inlet/outlet pipe (3) upstream of the valves; and the system (4) further comprises:
∘ at least one device for ensuring air flow through the said valves and inlet/outlet pipe, preferably a ventilator (43) or a pump;
∘ preferably at least one airflow measuring device (41'b, 42'b, 42"b) for measuring the air flow through the valve on the inlet/outlet pipe or through any valve;
∘ preferably a reservoir (44) for CO₂, where it is stored at the pressure of 1 to 5 bar, connected to the second pipe (42);
∘ an outlet pipe (5) for leading the used air from the chamber through the wall to the external environment of the building where the device is installed; and
- and **in that** the inlet/outlet pipe is coupled to the chamber (2) with a gate valve (7), which comprises:
∘ a rotating part (72);
∘ preferably a washer (73);
∘ a seal (74); and
∘ a static part (71) adapted for introduction into the inlet/outlet pipe;
∘ wherein the rotating part (72) has two removable parts, which are screwed into place from the interior of the chamber, the seal (74) is installed in the static part (71), while the preferred washer (73) prevents rotation of the chamber, when the rotating part (72) is screwed into place with the static part (71), and wherein the rotating part (72) and the static part (71) have corresponding treads, which can interlock so that air leakage is prevented.

2. The device according to claim 1, **characterized in that characterized in that** the CO₂ distributing system may have more pipes and valves, preferably one additional pipe with a valve is provided for delivering air to the chamber before it is filled with CO₂ in order to achieve CO₂ concentrations below 100%.

3. The device according to claim 1 or claim 2, **characterized in that** the device (1) may have one airflow measuring device (41'b, 42'b, 42"b) for measuring airflow through the inlet/outlet pipe (3) or several airflow measuring devices (41'b, 42'b, 42"b) installed on each pipe (41, 42, 5) of the CO₂ distribution system (4) for measuring airflow through the valve on each pipe (41, 42, 5).

4. The device according to any of the preceding claims, **characterized in that** the chamber (2) is for single use.

5. The device according to any of the preceding claims, **characterized in that** the chamber (2) is a flexible chamber such as a wrap or is designed as a chamber with supporting elements enabling certain geometry of the chamber when it is filled with air and/or CO₂, wherein the chamber may have any size and made of any biocompatible material not permeable to CO₂, the preferred choice is polyethylene, especially low-density polyethylene.

6. The device according to any of the preceding claims, **characterized in that** the device (1) is provided with the reservoir (44) for storing CO₂ at pressure from 1 to 5 bar, the reservoir (44) being connected to the gas tank (6), where the gas is stored at pressures around 50 bars.

7. The device according to any of the preceding claims, **characterized in that** the CO₂ distribution system (4) inside the housing is airtight, which is ensured by suitable seals for non-metal components and/or welding all metal components to each other, meaning that the valves are welded to the pipes, while the pipes are welded to the housing of the ventilator (43); and that the inlet/outlet pipe (3) is connected to the system with a clamp and all attachments are provided with suitable seals, so that the CO₂ does not leak into the room where the device (1) is used.

8. The device according to any of the preceding claims, **characterized in that** the valves may be any suitable, including electromagnetic or mechanic, controlled in any suitable way.

9. The device according to any of the preceding claims, **characterized in that** the outlet pipe (5) for leading the used air from the chamber through the wall to the external environment of the building where the device is installed is equipped with a telescopic transition for easy adjustment to a wide variety of walls, wherein the telescopic part (51) comprises:
- an inner part (511), the inner part's flange optionally provided with a seal for improved sealing;
- an outer part (512);
- a washer (513); and
- a nut (514) for tightening;
wherein the smaller inner part (511) can move along the outer part (512) and a threaded pole (511') runs through the entire length of the telescopic part (51).

10. The device according to any of the preceding claims, **characterized in that** it is further provided with a filter in the CO₂ distribution system (4) to filter out any impurities and/or a silencer for decreasing the sound resulting from the flow of gas under pressure, wherein said filter is preferably installed in the pipe of the CO₂ distribution system between the ventilator (43) and the inlet/outlet pipe (3) and the silencer is preferably installed between the second valve and the ventilator.

11. The device according to any of the preceding claims, **characterized in that** it is further equipped with suitable electronics for easier controlling and managing of the device, wherein the controller has suitable buttons connected with a control device that opens and closes the valves used in the CO₂ distribution system (4), so that any particular CO₂ concentration in the chamber may be achieved.

12. The device according to any of the preceding claims for use in therapy, especially in therapy of patients with impaired microcirculation; especially but not limited to patients affected with: chronic and acute wounds, neuropathy, muscle tears, etc.

13. The device according to the preceding claim, **characterized in that** the preferred range of CO₂ concentration inside the chamber is 10 to 100 %.

14. The device according to any of the preceding claims, arranged to allow the following steps:
- in case of flexible chambers, the chamber is attachable to the inlet-outlet pipe via the valve or the gate valve and is arranged to allow placing at least a part of patient's body into the chamber and sealing the chamber;
- sucking out all air from the chamber with the first valve with a ventilator and leading all sucked air to the exterior of the building with the room where the device is used;
- leading CO₂ from the tank to the CO₂ distribution system or preferably the reservoir for allowing the CO₂ to expand in the reservoir, which results in decrease of its pressure in an increase of its temperature;
- closing the first valve and opening the second valve to supply a desired amount of air into the chamber and/or opening the third valve to supply a desired amount of 100% CO₂ into the chamber; and
- when the use of the device (1) is finished, the air with the CO₂ is sucked from the chamber (2) through the outlet pipe (5) and led outside of the building where the device (1) is installed.

## Patentansprüche

1. Vorrichtung (1) zur transkutanen Anwendung von Kohlendioxid zur Behandlung von chronischen Wunden oder Neuropathie, umfassend mindestens:
- eine therapeutische Kammer (2), die mindestens einen Teil umfasst, um ein Körperteil eines Patienten aufzunehmen, an dem das Kohlendioxid angewendet werden soll,
- ein CO₂-Verteilungssystem (4), umfassend
∘ ein erstes Rohr (41) mit einem ersten Ventil (41a) zum Zuführen von CO₂ aus einem Tank (6) oder einem Reservoir,
∘ ein zweites Rohr (42) mit einem zweiten Ventil (42a) zum Absaugen von Luft aus der Kammer (2);
- mindestens einen Tank (6) zur Speicherung von CO₂, der in geeigneter Weise mit dem CO₂-Verteilungssystem (4) oder vorzugsweise mit einem Reservoir (44) verbunden ist,
**dadurch gekennzeichnet, dass** die Vorrichtung (1) ferner umfasst:
- ein Einlass-/Auslassrohr (3), das die Kammer (2) mit dem CO₂-Verteilungssystem verbindet, wobei das Einlass-/Auslassrohr (2) durch ein geeignetes Element oder ein Ventil (7) mit der Kammer verbunden ist;
- und dass das CO₂-Verteilungssystem (4) ein Gehäuse umfasst:
wobei das erste und das zweite Rohr in das Einlass-/Auslassrohr (3) vorgelagert vor die Ventile kombiniert sind; und das System (4) ferner umfasst:
∘ mindestens eine Einrichtung zur Gewährleistung einer Luftströmung durch die genannten Ventile und ein Ein-/Auslassrohr, vorzugsweise einen Ventilator (43) oder eine Pumpe;
∘ vorzugsweise mindestens eine Luftstrommesseinrichtung (41'b, 42'b, 42"b) zur Messung des Luftstroms durch das Ventil am Einlass-/Auslassrohr oder durch ein beliebiges Ventil;
∘ vorzugsweise ein Reservoir (44) für CO₂, wobei es bei einem Druck von 1 bis 5 bar gelagert wird, das mit dem zweiten Rohr (42) verbunden ist;
o ein Auslassrohr (5) zum Führen der gebrauchten Luft aus der Kammer durch die Wand in die äußere Umgebung des Gebäudes, in dem die Vorrichtung installiert ist;
- und dass das Einlass-/Auslassrohr mit der Kammer (2) mit einem Absperrschieber (7) gekoppelt ist, das umfasst:
∘ ein Drehteil (72);
∘ vorzugsweise eine Unterlegscheibe (73);
∘ eine Dichtung (74); und
∘ ein statisches Teil (71), das zum Einführen in das Einlass-/Auslassrohr eingerichtet ist;
∘ wobei das Drehteil (72) zwei abnehmbare Teile aufweist, die im Innenraum der Kammer verschraubt sind, wobei die Dichtung (74) im statischen Teil (71) angeordnet ist, während die bevorzugte Unterlegscheibe (73) eine Drehung der Kammer verhindert, wenn das Drehteil (72) mit dem statischen Teil (71) in Position gebracht wird, und wobei das Drehteil (72) und das statische Teil (71) entsprechende Profile aufweisen, die einrasten können, so dass ein Luftverlust verhindert wird.

2. Vorrichtung nach Anspruch 1, **dadurch gekennzeichnet, dass** das CO₂-Verteilungssystem mehrere Rohre und Ventile aufweisen kann, wobei vorzugsweise ein zusätzliches Rohr mit einem Ventil zur Luftzufuhr in die Kammer vorgesehen ist, bevor diese mit CO₂ gefüllt wird, um CO₂-Konzentrationen unter 100% zu erreichen.

3. Vorrichtung nach Anspruch 1 oder 2, **dadurch gekennzeichnet, dass** die Vorrichtung (1) eine Luftstrommessvorrichtung (41'b, 42'b, 42"b) zum Messen des Luftstroms durch das Einlass-/Auslassrohr (3) oder mehrere Luftstrommessvorrichtungen (41'b, 42'b, 42"b), die an jedem Rohr (41, 42, 5) des CO₂-Verteilungssystems (4) angeordnet sind, zum Messen des Luftstroms durch das Ventil an jedem Rohr (41, 42, 5) aufweisen kann.

4. Vorrichtung nach einem der vorhergehenden Ansprüche, **dadurch gekennzeichnet, dass** die Kammer (2) zum Einmalgebrauch ist.

5. Vorrichtung nach einem der vorhergehenden Ansprüche, **dadurch gekennzeichnet, dass** die Kammer (2) eine flexible Kammer, wie eine Umhüllung, ist oder als Kammer mit Stützelementen ausgebildet ist, die eine gewisse Geometrie der Kammer ermöglichen, wenn sie mit Luft und/oder CO₂ gefüllt ist, wobei die Kammer eine beliebige Größe aufweisen kann und aus einem beliebigen, für CO₂ nicht durchlässigen biokompatiblen Material hergestellt ist, wobei die bevorzugte Wahl Polyethylen, insbesondere Polyethylen niedriger Dichte, ist.

6. Vorrichtung nach einem der vorhergehenden Ansprüche, **dadurch gekennzeichnet, dass** die Vorrichtung (1) mit dem Reservoir (44) zum Speichern von CO₂ bei einem Druck von 1 bis 5 bar versehen ist, wobei das Reservoir (44) mit dem Gastank (6) verbunden ist, wobei das Gas bei Drücken um 50 bar gelagert wird.

7. Vorrichtung nach einem der vorhergehenden Ansprüche, **dadurch gekennzeichnet, dass** das CO₂-Verteilungssystem (4) innerhalb des Gehäuses luftdicht ist, was durch geeignete Dichtungen für Nicht-Metall-Komponenten und/oder das Verschweißen aller Metallkomponenten miteinander gewährleistet wird, was bedeutet, dass die Ventile mit den Rohren verschweißt werden, während die Rohre mit dem Gehäuse des Ventilators (43) verschweißt werden; und dass das Einlass-/Auslassrohr (3) mit einer Klemme mit dem System verbunden ist und alle Verbindungsteile mit geeigneten Dichtungen versehen sind, so dass das CO₂ nicht in den Raum entweicht, in dem die Vorrichtung (1) verwendet wird.

8. Vorrichtung nach einem der vorhergehenden Ansprüche, **dadurch gekennzeichnet, dass** die Ventile beliebig sein können, einschließlich elektromagnetische oder mechanische, die auf jede geeignete Weise gesteuert werden können.

9. Vorrichtung nach einem der vorhergehenden Ansprüche, **dadurch gekennzeichnet, dass** das Auslassrohr (5) zum Führen der gebrauchten Luft aus der Kammer durch die Wand zur Äußeren Umgebung des Gebäudes, in dem die Vorrichtung angeordnet ist, mit einem teleskopischen Übergang zur leichteren Anpassung an eine hohe Anzahl von Wänden ausgestattet ist, wobei das Teleskopteil (51) umfasst:
- ein Innenteil (511), wobei der Flansch der Innenteile gegebenenfalls mit einer Dichtung zur besseren Abdichtung versehen ist;
- ein Außenteil (512);
- eine Unterlegscheibe (513); und
- eine Mutter (514) zum Festziehen;
wobei sich das kleinere Innenteil (511) entlang des Außenteils (512) bewegen kann und eine Gewindestange (511') über die gesamte Länge des Teleskopteils (51) verläuft.

10. Vorrichtung nach einem der vorhergehenden Ansprüche, **dadurch gekennzeichnet, dass** sie ferner mit einem Filter in dem CO₂-Verteilungssystem (4) versehen ist, um jegliche Verunreinigungen herauszufiltern und/oder einem Schalldämpfer zur Verringerung des aus dem Gasstrom resultierenden Schalls, wobei der Filter vorzugsweise im Rohr des CO₂-Verteilungssystems zwischen dem Ventilator (43) und dem Einlass-/Auslassrohr (3) angeordnet ist und der Schalldämpfer vorzugsweise zwischen dem zweiten Ventil und dem Ventilator angeordnet ist.

11. Vorrichtung nach einem der vorhergehenden Ansprüche, **dadurch gekennzeichnet, dass** sie ferner mit einer geeigneten Elektronik zur leichteren Steuerung und Handhabung der Vorrichtung ausgestattet ist, wobei die Steuerung geeignete Tasten aufweist, die mit einer Steuervorrichtung verbunden sind, die die in dem CO₂-Verteilungssystem (4) verwendeten Ventile öffnet und schließt, so dass eine bestimmte CO₂-Konzentration in der Kammer erreicht werden kann.

12. Vorrichtung nach einem der vorhergehenden Ansprüche zur Verwendung in der Therapie, insbesondere bei der Therapie von Patienten mit beeinträchtigter Mikrozirkulation, vorzugsweise aber nicht ausschließlich für Patienten, mit chronischen und akuten Wunden, Neuropathien, Muskelrissen usw.

13. Vorrichtung nach dem vorhergehenden Anspruch, **dadurch gekennzeichnet, dass** der bevorzugte Bereich der CO₂-Konzentration innerhalb der Kammer 10 bis 100% beträgt.

14. Vorrichtung nach einem der vorhergehenden Ansprüche, die eingerichtet ist, um die folgenden Schritte zu ermöglichen:
- wobei bei flexiblen Kammern, die Kammer über das Ventil oder das Absperrventil an dem Einlass-Auslassrohr befestigt wird und eingerichtet ist, um zumindest einen Teil des Körpers des Patienten in der Kammer zu platzieren und die Kammer abzudichten;
- Absaugen der gesamten Luft aus der Kammer mit dem ersten Ventil mit einem Ventilator und Ausführen der abgesaugten Luft durch die Außenseite des Gebäudes mit dem Raum, in dem die Vorrichtung verwendet wird;
- Zuführen des CO₂ aus dem Tank zum CO₂-Verteilungssystem oder vorzugsweise dem Reservoir, um es dem CO₂ zu ermöglichen, sich in dem Reservoir auszudehnen, was bei einer Temperaturerhöhung zur Verringerung seines Drucks führt;
- Schließen des ersten Ventils und Öffnen des zweiten Ventils, um eine gewünschte Menge an Luft in die Kammer einströmen zu lassen und/oder Öffnen des dritten Ventils, um die gewünschte Menge von 100% CO₂ in die Kammer strömen zu lassen; und
- nach Abschluss der Verwendung der Vorrichtung (1) wird die Luft mit dem CO₂ aus der Kammer (2) durch das Auslassrohr (5) abgesaugt und außerhalb des Gebäudes, in dem die Vorrichtung (1) angeordnet ist, abgeleitet.

## Revendications

1. Dispositif (1) pour l'application transcutanée de dioxyde de carbone pour le traitement de plaies chroniques ou de neuropathie comprenant au moins :
- une chambre thérapeutique (2) comprenant au moins une partie destinée à recevoir une partie du corps d'un patient, à laquelle le dioxyde de carbone doit être appliqué,
- un système de distribution de CO₂ (4), comprenant
∘ un premier tuyau (41) avec une première soupape (41a) pour fournir du CO₂ à partir d'un récipient (6) ou d'un réservoir,
∘ un second tuyau (42) avec une seconde soupape (42a) pour l'aspiration de l'air hors de la chambre (2) ;
- au moins un récipient (6) pour stocker du CO₂ relié de manière appropriée au système de distribution de CO₂ (4) ou de préférence à un réservoir (44),
**caractérisé en ce que** le dispositif (1) comprend en outre :
- un tuyau d'entrée/sortie (3) reliant la chambre (2) à un système de distribution de CO₂, le tuyau d'entrée/sortie étant relié à la chambre (2) avec un élément approprié ou une soupape (7) ;
- et **en ce que** le système de distribution de CO₂ (4) comprend un boîtier :
les premier et second tuyaux étant combinés dans le tuyau d'entrée/sortie (3) en amont des soupapes ; et le système (4) comprenant en outre :
∘ au moins un dispositif pour assurer un écoulement d'air à travers lesdites soupapes et ledit tuyau d'entrée/sortie, de préférence un ventilateur (43) ou une pompe ;
∘ de préférence au moins un dispositif de mesure de l'écoulement d'air (41'b, 42'b, 42"b) pour mesurer l'écoulement d'air à travers la soupape sur le tuyau d'entrée/sortie ou à travers une quelconque soupape ;
∘ de préférence un réservoir (44) pour le CO₂, où ce dernier est stocké à la pression de 1 à 5 bars, relié au second tuyau (42) ;
∘ un tuyau de sortie (5) pour amener l'air vicié depuis la chambre à travers la paroi vers l'environnement externe du bâtiment où le dispositif est installé ; et
- et **en ce que** le tuyau d'entrée/sortie est couplé à la chambre (2) avec une vanne d'arrêt (7), qui comprend :
∘ une partie rotative (72) ;
∘ de préférence une rondelle (73) ;
∘ un joint d'étanchéité (74) ; et
∘ une partie statique (71) conçue pour être introduite dans le tuyau d'entrée/sortie ;
∘ la partie rotative (72) ayant deux parties amovibles, qui sont vissées en place à partir de l'intérieur de la chambre, le joint d'étanchéité (74) étant installé dans la partie statique (71), tandis que la rondelle préférée (73) empêche la rotation de la chambre, lorsque la partie rotative (72) est vissée en place avec la partie statique (71), et la partie rotative (72) et la partie statique (71) ayant des surfaces de roulement correspondantes, qui peuvent se verrouiller de manière à empêcher toute fuite d'air.

2. Dispositif selon la revendication 1, **caractérisé en ce que** le système de distribution de CO₂ peut avoir plus de tuyaux et de soupapes, de préférence un tuyau supplémentaire avec une soupape est prévu pour distribuer de l'air à la chambre avant qu'il soit rempli de CO₂ afin d'obtenir des concentrations de CO₂ inférieures à 100 %.

3. Dispositif selon la revendication 1 ou la revendication 2, **caractérisé en ce que** le dispositif (1) peut avoir un dispositif de mesure d'écoulement d'air (41'b, 42'b, 42"b) pour mesurer l'écoulement d'air à travers le tuyau d'entrée/sortie (3) ou plusieurs dispositifs de mesure d'écoulement d'air (41'b, 42'b, 42"b) installés sur chaque tuyau (41, 42, 5) du système de distribution de CO₂ (4) pour mesurer l'écoulement d'air à travers la soupape sur chaque tuyau (41, 42, 5).

4. Dispositif selon l'une quelconque des revendications précédentes, **caractérisé en ce que** la chambre (2) est à usage unique.

5. Dispositif selon l'une quelconque des revendications précédentes, **caractérisé en ce que** la chambre (2) est une chambre flexible telle qu'une enveloppe ou est conçue sous la forme d'une chambre avec des éléments de support permettant une certaine géométrie de la chambre lorsqu'elle est remplie d'air et/ou de CO₂, la chambre pouvant avoir n'importe quelle taille et être constituée de n'importe quel matériau biocompatible non perméable au CO₂, le choix préféré étant du polyéthylène, en particulier du polyéthylène basse densité.

6. Dispositif selon l'une quelconque des revendications précédentes, **caractérisé en ce que** le dispositif (1) est pourvu du réservoir (44) pour stocker du CO₂ à une pression de 1 à 5 bars, le réservoir (44) étant relié au récipient de gaz (6), le gaz étant stocké à des pressions autour de 50 bars.

7. Dispositif selon l'une quelconque des revendications précédentes, **caractérisé en ce que** le système de distribution de CO₂ (4) à l'intérieur du boîtier est étanche à l'air, ce qui est assuré par des joints adaptés pour des composants non métalliques et/ou le soudage de tous les composants métalliques l'un à l'autre, ce qui signifie que les soupapes sont soudées aux tuyaux, tandis que les tuyaux sont soudés au boîtier du ventilateur (43) ; et **en ce que** le tuyau d'entrée/sortie (3) est relié au système avec une pince et toutes les fixations sont pourvues de joints d'étanchéité appropriés, de telle sorte que le CO₂ ne fuit pas dans la pièce où le dispositif (1) est utilisé.

8. Dispositif selon l'une quelconque des revendications précédentes, **caractérisé en ce que** les soupapes peuvent être appropriées à tout, y compris électromagnétiques ou mécaniques, commandées de toute manière appropriée.

9. Dispositif selon l'une quelconque des revendications précédentes, **caractérisé en ce que** le tuyau de sortie (5) pour conduire l'air vicié de la chambre à travers la paroi vers l'environnement externe du bâtiment où le dispositif est installé est équipé d'une transition télescopique pour un ajustement facile à une grande variété de parois, la partie télescopique (51) comprenant :
- une partie interne (511), la bride de la partie interne étant éventuellement pourvue d'un joint d'étanchéité pour améliorer l'étanchéité ;
- une partie externe (512) ;
- une rondelle (513) ; et
- un écrou (514) de serrage ;
la partie interne plus petite (511) pouvant se déplacer le long de la partie externe (512) et une tige filetée (511 ') s'étendant à travers toute la longueur de la partie télescopique (51).

10. Dispositif selon l'une quelconque des revendications précédentes, **caractérisé en ce qu'**il est en outre pourvu d'un filtre dans le système de distribution de CO₂ (4) pour filtrer toute impureté et/ou un silencieux pour diminuer le son résultant de l'écoulement de gaz sous pression, ledit filtre étant de préférence installé dans le tuyau du système de distribution de CO₂ entre le ventilateur (43) et le tuyau d'entrée/sortie (3) et le silencieux étant de préférence installé entre la seconde soupape et le ventilateur.

11. Dispositif selon l'une quelconque des revendications précédentes, **caractérisé en ce qu'**il est en outre équipé d'une électronique appropriée pour commander et gérer plus facilement le dispositif, le régulateur ayant des boutons appropriés reliés à un dispositif de commande qui ouvre et ferme les soupapes utilisées dans le système de distribution de CO₂ (4), de telle sorte qu'il est possible d'obtenir toute concentration en CO₂.

12. Dispositif selon l'une quelconque des revendications précédentes destiné à être utilisé en thérapie, en particulier dans la thérapie de patients atteints de microcirculation altérée ; en particulier, mais sans s'y limiter, des patients affectés par : des plaies chroniques et aiguës, une neuropathie, des déchirures musculaires, etc.

13. Dispositif selon la revendication précédente, **caractérisé en ce que** la plage préférée de concentration en CO₂ à l'intérieur de la chambre est de 10 à 100 %.

14. Dispositif selon l'une quelconque des revendications précédentes, agencé pour permettre les étapes suivantes :
- dans le cas de chambres flexibles, la chambre peut être fixée au tuyau d'entrée-sortie par l'intermédiaire de la soupape ou de la vanne d'arrêt et est conçue pour permettre le placement d'au moins une partie du corps du patient dans la chambre et l'étanchéification de la chambre ;
- l'aspiration de la totalité de l'air de la chambre avec la première soupape avec un ventilateur et l'acheminement de l'air aspiré vers l'extérieur du bâtiment accueillant la pièce où le dispositif est utilisé ;
- la conduite du CO₂ du récipient au système de distribution de CO₂ ou de préférence du réservoir pour permettre au CO₂ de se dilater dans le réservoir, ce qui conduit à une diminution de sa pression lors d'une augmentation de sa température ;
- la fermeture de la première soupape et l'ouverture de la seconde soupape pour fournir une quantité souhaitée d'air dans la chambre et/ou l'ouverture de la troisième soupape pour fournir une quantité souhaitée de CO₂ à 100 % dans la chambre ; et
- lorsque l'utilisation du dispositif (1) est terminée, l'air avec le CO₂ est aspiré de la chambre (2) à travers le tuyau de sortie (5) et conduit à l'extérieur du bâtiment où le dispositif (1) est installé.
